# NEW EUROPEAN PATENT SPECIFICATION

(11) **EP 0 249 983 B2**
(45) Date of publication and mention of the opposition decision: **19.03.1997**
(45) Mention of the grant of the patent: 20.10.1993
(21) Application number: 87108799.5
(22) Date of filing: 19.06.1987
(51) Int. Cl.: G01N 33/543, G01N 33/563

(54) **Reagent kit for use in sandwich immunoassay**
Reagenz-Kit zur Verwendung in Sandwich-Immunotesten
Trousse de réactifs pour utilisation dans des immuno-essais sandwichs

(30) Priority: 20.06.1986 JP 142885/86; 03.04.1987 JP 82512/87; 16.06.1987 JP 149324/87
(43) Date of publication of application: 23.12.1987
(73) Proprietor: Kabushiki Kaisha Meidensha, Shinagawa-ku Tokyo 141 (JP)
(72) Inventor: AOYAGI, Shigeo, Shinjuku-ku Tokyo (JP); KUSUMI, Miyoko, Koganei-shi, Tokyo (JP); MATSUYUKI, Akira, Sumida-ku, Tokyo (JP); OSHIMA, Nobuo, Ohta-ku, Tokyo (JP)
(74) Representative: Dipl.-Phys.Dr. Manitz Dipl.-Ing. Finsterwald Dipl.-Ing. Grämkow Dipl.Chem.Dr. Heyn Dipl.Phys. Rotermund Morgan, B.Sc.(Phys.)

(56) References cited:
- EP-A- 0 083 869
- EP-A- 0 116 454
- EP-A- 0 125 893
- WO-A-85/00663
- US-A- 4 002 532
- US-A- 4 414 324
- JOURNAL OF IMMUNOLOGICAL METHODS, vol. 83, no. 2, 1985, pages 327-336, Elsevier Science Publishers B.V., Amsterdam, NL; H. TANIMORI et al.: "A new solid support for sandwich enzyme immunoassays of human immunoglobulin G"
- Behringwerke AG. Packungsbeilage Enzygnost IgE. Juni 1985.
- Schuurs, Van Weemen. Clinica Chimica Acta. 81 pp. 1-40. 1977.
- Monroe. Analytical Chemistry, 56 pp. 920A-931A. 1984.

## Description

### BACKGROUND OF THE INVENTION

This invention relates to sandwich immunoassays utilizing immunoreaction for measuring an extremely small quantity of an ingredient included in blood or other organic samples and, more particularly to an improved combination of reagents for use in such sandwich immunoassays to increase the accuracy, of measurement of the ingredient.

Immunoassays have been used widely for measuring an extremely small quantity of an ingredient included in blood or other organic samples by utilizing immunoreaction or antigen-antibody reaction. Such immunoassays are generally of the two types; the "competitive immunoassay" and the "non-competitive immunoassay". The competitive immunoassay employs a predetermined quantity of labeled antigen when binds to a predetermined quantity of antibody in a competitive manner with target antigen. The quantity of the target antigen is measured from the quantity of the labeled antigen binding to the antibody or the quantity of the labeled antigen which does not bind to the antibody. In the competitive immunoassay, however, the labeled antigen is required to have a high concentration for its reaction to the antibody. In addition, the competitive immunoassay exhibits a poor measurement sensitivity as low as about a twentieth of the quantity of the labeled antigen.

The non-competitive immunoassay is represented by a sandwich immunoassay which employs a solid phase coated on its surface with an excessive quantity of insolubilized antibody to trap a target antigen to be measured. The solid phase is washed and then incubated with an excessive amount of labeled antibody so that the labeled antibody can react with the trapped target antigen. The excessive labeled antibody is washed out away from the solid phase. The quantity of the target antigen is measured from the quantity of the label provided on the labeled antibody binding to the antigen. Although the sandwich immunoassay can provide a high measurement sensitivity, there is a tendency of non-specific binding of the labeled antibody to the solid phase, causing a considerable reduction in the sensitivity and measurable range of the sandwich immunoassay.

### SUMMARY OF THE INVENTION

Therefore, a main object of the invention is to reduce the degree of non-specific binding of the labeled antibody to the solid phase so as to increase the accuracy of the sandwich immunoassay.

It is another object of the invention to provide a kit for use in a sandwich immunoassay to reduce the degree of non-specific binding of the labeled antibody to the solid phase to a considerable extent.

It is still another object of the invention to provide a kit for use in a sandwich immunoassay to increase the sensitivity and measurable range of the sandwich immunoassay.

It is still another object of the invention to provide a kit for use in a sandwich immunoassay to reduce the degree of deviation of sandwich immunoassay.

There is provided, in accordance with the invention, a kit for use in a sandwich immunoassay employing labeled and solid-phase antibodies reactive with an antigen in the presence of a reaction solution to bind the antigen between the labeled and solid-phase antibodies, said kit containing a solid-phase antibody bound to a solid phase, a labeled antibody having a label enzyme bound to the antibody and a reaction solution, and being characterized in that the labeled antibody is originated from a rabbit, the solid-phase antibody is originated from a goat and the label enzyme is glucose oxidase.

### BRIEF DESCRIPTION OF THE DRAWINGS

This invention will be described in greater detail by reference to the following description taken in connection with the accompanying drawings, in which:
Fig. 1 is a graph showing the relationship between chemiluminescent intensity and AFP quantity;
Fig. 2 is a schematic diagram showing the lgG structure;
Fig.3 is a graph plotting non-specific binding percentage with respect to given examples; and
Fig. 4 is a graph showing the relationship between chemiluminescent intensity and AFP quantity.

### DETAILED DESCRIPTION OF THE INVENTION

One embodiment of the invention will be described. A target antigen is assayed by a sandwich enzyme immunoassay (EIA) technique employing first and second antibodies reactive in a specific manner with the target antigen in the presence of a reaction solution to trap the target antigen between the first and second antibodies. The antibodies are obtained from serum drawn from a selected animal into which the target antigen is injected. The first antibody is referred to as a solid-phase antibody which is coated on a solid phase such as polystyrene balls. The second antibody is referred to as a labeled antibody which has a label enzyme bound thereon. The label enzyme is glucose oxidase. A chemiluminescence technique is. used to measure the quantity of the label enzyme which corresponds to the quantity of the target antigen. These procedures will be decribed in greater detail on an assumption that the target antigen is α-fetoprotein (AFP) and the label enzyme is glucose oxidase (GOD). The reaction solution used is 0.056 mol/l sodium phosphate buffer (pH 6.3) containing 0.1% sodium azide, 0.2% bovine serum albumin (BSA) and 0.337% NaCI. This reaction solution is referred to as reaction solution A.

First of all, AFP is injected six times under the back skin of selected one of animals including rabbit, goat and the like at intervals of one week. On the seventh week, a booster is injected into the selected animal. After five days, a proper amount of blood is drawn from the selected animal.

Anti-AFP immunoglobulin G (IgG) is purified from the drawn serum in the following manner. First of all, 2 ml saturated ammonium sulfate is dripped into 2 ml of serum. The resulting solution is agitated slowly at a temperature of 4°C for two or three hours. The resulting cloudy solution is then placed in a centrifugation tube. Centrifugation is performed at a temperature of 4°C with a contrifugal separator rotating at a speed of 3000 rpm for 15 minutes. The supernatuant is removed from the centrifugation tube, whereas the precipitate is dissolved with 2 ml of 0.1 mol/l sodium phosphate buffer (pH 6.0) containing 5 mmol/l ethylenediaminetetraacetic acid (EDTA). The resulting solution is eluted at a flow rate of 6 ml/hour on s Sephacryl S-300 (a trademark of Pharmacia Fine Chemicals, Sweden) column (1 x 90 cm) equilibrated with 0.1 mol/l sodium phosphate buffer (pH 6.0) containing 5 mmol/l ethylenediaminetetraacetic acid (EDTA). The eluted solution is divided into 1 ml of samples. The lgG fractions, which absorb light at 280 nm, are gathered from the respective samples.

The resulting anti-AFP IgG is coated through physical absorption on a solid phase by soaking a polystyrene ball (6.5 mm in diameter) available from Ichiko Co., Japan in 0.1 mol/l sodium phosphate buffer (pH 7.5) containing 0.1 mg/ml anti-AFP IgG all the night through at a temperature of 4 °C. The IgG-coated polystyrene ball is washed three times in 0.1 mol I sodium phosphate buffer (pH 7.0) and then washed three times with 0.01 mol/l sodium phosphate buffer (pH 7.0) containing 0.1 mol/l NaCI 0.1% bovine serum albumin (BSA) and 0.1% NaN₃. The washed polystyrene ball is stored at a temperature of 4 °C in 0.01 molil sodium phosphate buffer (pH 7.0) containing 0.1 mol/l NaCI, 0.1% bovine serum albumin (BSA) and 0.1% NaN₃.

The labeled antibody, anti-AFP IgG-GOD, is prepared through the following three steps:
(1) Maleimide glucose oxidase (Maleimide GOD) is prepared as follows. Succinimidyl-4-maleimidobutyrate (GMBS) is added to about 3 mg of glucose oxidase (GOD) placed in each of four test tubes containing 0.3 ml of 0.1 mol I sodium phosphate buffer (pH 7.0) to produce a solution having a GOD/GMBS ratio of 1/50. This solution is incubated at a temperature of 30 °C for one hour. The resulting solution is desalted at a flow rate of 12 ml/hour on a Sephadex G-25 (a trademark of Pharmacia Fine Chemicals, Sweden) column (1 x 30 cm) equilibrated with 0.1 mol/l sodium phosphate buffer (pH 6.0). The desalted solution is divided into 1 ml of maleimido GOD samples.
(2) The sulfhydrated anti-AFP immunoglobulin G (SH-IgG) is prepared as follows. First of all, 2 ml of IgG solution containing 10 mg of anti-AFP IgG, 0.1 mol/l sodium phosphate buffer (pH 6.0) and 5 mmol/l ethylenediaminetetraacetic acid (EDTA) is prepared. The IgG solution is added to a solution containing S-acetylmercaptosuccimic anhydride (ASMSa) dissolved in N, N-dimethylformamide (DMF) to produce a solution having an IgG/ASMSa ratio, by mol concentration, of 1/300. The resulting solution is agitated at a temperature of 30°C for 30 minutes. In succession, this solution is added with 0.1 ml of 0.1 mol/l Tris-HCλ buffer (pH 7.0), 0.02 ml of 0.1 mol I ethylenediaminetetraacetic acid (EDTA) (pH 7.0) and 0.1 ml of 1 mol/l hydroxylamine hydrochloride (pH 7.0). The resulting solution is incubated at a temperature of 30°C for 4 minutes and then the resulting solution is desalted at a flow rate of 12 ml/hour on a Sephadex G-25 (a trademark of Pharmacia Fine Chemicals, Sweden) column (1x30 cm) equilibrated with 0.1 mol/l sodium phosphate buffer (pH 6.0) containing 5 mmol/l ethylenediaminetetraacetic acid (EDTA). The desalted solution is divided into 1 ml of SH-IgG samples. These SH-IgG samples are concentrated in an ice-cooled collodion bag.
(3) The labeled antibody was prepared as follows. The maleimido GOD is added to the SH-IgG solution with the same mol concentration as the maleimide GOD. After the resulting solution is held one hour at a temperature of 30°C and then held at a temperature of 4°C stationary all the night through, it is eluted at a flow rate of 6 ml/hour on a Sephacryl S-300 (a trademark of Pharmacia Fine Chemicals, Sweden) column (1 x 90 cm) equilibrated with 0.1 mol/l sodium phosphate buffer solution (pH 6.5) containing 5 mmol/l ethylenediaminetetraacetic acid (EDTA). The eluted solution is divided into 1 ml of samples. The anti-AFP IgG-GOD (labeled antibody) is gathered from the respective samples and stored at a temperature of 4°C in a solution containing 0.1% NaN3 and 0.1% bovine serum albumin (BSA).

The labeled antibody (anti-AFP IgG-GOD) is bound through the antigen (AFP) to the solid-phase antibody (anti-AFP IgG) in the presence of the reaction solution A (0.056 mol/l sodium phosphate buffer (pH 6.3) containing 0.1% sodium azide, 0.2% bovine serum albumin (BSA) and 0.337% NaCI) in the following manner. The anti-AFP IgG-coated polystyrene ball is incubated at room temperature with AFP (antigen) diluted with the reaction solution A in an assay tube for six hours. The polystylene ball is then washed three times with distilled water. The washed polystyrene ball is incubated at room temperature with anti-AFP IgG-GOD (labeled antibody) in the reaction solution A all the night through. The polystyrene ball is then washed again with distilled water and transferred into another clean assay tube.

A chemiluminescent technique is used to determine the quantity of the label enzyme; that is, the quantity of the antigen. First of all, 0.3 ml of 0.01 mol/l acetate buffer (pH 5.1) containing mol/l glucose is added to the assay tube. The assay tube is held stationary at a temperature of 37°C for two hours. The resulting solution is taken to prepare a 0.1 ml sample. The sample is added with 0.5 ml of 0.2 mol/l carbonate buffer (pH 9.8) containing 2 x 10⁻⁷ mol/l luminol and then 0.5 ml of 6 x 10⁻³ mol/l potassium ferricyanide. The concentration of the resulting hydrogen peroxide is measured from the light emitted upon chemiluminescent reaction of the luminol and potassium ferricyanide and the hydrogen peroxide by a Luminometer UPD-8000 (a trademark of Meidensha Electric Mfg., Co., Japan) which starts its operation 15 second after the luminol and potassium ferricyanide are added to the assay tube and counts the light emitted for 30 seconds.

Although the antigen has been described in connection with α-fetoprotein (AFP), it is to be noted that it may be anti-carcinoembryonic antigen (CEA) or the like. Anti-CEA IgG is purified from serum drawn from selected one of animals including, rabbit, goat and the like into which CEA is injected six times at intervals of one week and a booster is injected on the seventh week.

A serious problem involved with such sandwich enzyme immunoassay (EIA) is the tendency of non-specific binding of a relatively great quantity of labeled antibody to the solid phase, degrading the accuracy of sandwich enzyme immunoassay. The inventors found that the degree of non-specific binding of the labeled antibody to the solid phase was reduced when the labeled antibody was prepared from serum originated from rabbit, the solid-phase antibody was prepared from serum originated from goat and the label enzyme was glucose oxidase. A large number of tests were conducted to prove the effective combinations of the solid-phase and labeled antibodies.

The degree of non-specific binding of the labeled antibody to the solid phase was determined. For this purpose, an IgG-coated polystyrene ball was incubated at room temperature with IgG-GOD diluted hundredfold with 0.3 ml of reaction solution A in an assay tube all the night through. The polystyrene ball was washed three times with the reaction solution A and then transferred into another clean assay tube. Following this, 0.3 ml of 0.01 mol/l acetate buffer (pH 5.1) containing 0.5 mol/l glucose was added to the assay tube. The assay tube was held stationary at a temperature of 37°C for two hours. The resulting solution was taken to prepare a 0.1 ml sample. The sample was added with 0.5 ml of 0.2 mol/l carbonate buffer (pH 9.8) containing 2x10⁻⁷ mol/l luminol and then with 0.5 ml of 6x10⁻³ mol/l potassium ferricyanide. The light emitted upon chemiluminescent reaction of the luminol and potassium ferricyanide and the resulting hydrogen peroxide was measured by a Luminometer UPD-8000 which started its operation 15 seconds after the luminol and potassium ferricyanide were added to the assay tube and counted the light emitted for 30 seconds. The degree of non-specific binding of the labeled antibody to the solid phase was represented by the non-specific bonding ratio given as C1/C2 x 100 (%) where C1 is the number of counts measured for the labeled antibody binding to the polystyrene ball and C2 is the number of counts corresponding to the whole amount of the labeled antibody used in the assay tube.

Example 1 - The labeled antibody used was rabbit anti-AFP lgG-GOD and the solid-phase antibody used was goat anti-AFP IgG. The rabbit anti-AFP IgG was Nordic Co., Lot No. 16-184. A goat anti-AFP IgG-coated polystyrene ball was incubated with the rabbit anti-AFP IgG-GOD in the presence of the reaction solution A mixed with guinea pig serum.

Example 2 - The labeled antibody used was normal rabbit IgG-GOD and the solid-phase antibody used was normal goat IgG. The normal rabbit IgG was Miles Co., Lot No. 0019. The normal goat IgG was Cappel Co., Lot No. 24511. A normal goat IgG-coated polystyrene ball was incubated with the normal rabbit IgG-GOD in the presence of the reaction solution A mixed with guinea pig serum.

For comparison of the non-specific binding reduction effect obtainable by the invention, tests were conducted for the following comparative examples:
Comparative Example 1 - The labeled antibody used was goat anti-AFP IgG-GOD and the solid-phase antibody used was goat anti-AFP IgG. A goat anti-AFP IgG-coated polystyrene ball was incubated with the goat anti-AFP IgG-GOD in the presence of the reaction solution A mixed with guinea pig serum.
Comparative Example 2 - The labeled antibody used was rabbit anti-AFP IgG-GOD and the solid-phase antibody used was rabbit anti-AFP IgG. A rabbit anti-AFP IgG-coated polystyrene ball was incubated with the rabbit anti-AFP IgG-GOD in the presence of the reaction solution A mixed with guinea pig serum.
Comparative Example 3 - The labeled antibody used was normal goat IgG-GOD and the solid-phase antibody used was normal goat IgG. A normal goat IgG-coated polystyrene ball was incubated with the normal goat IgG-GOD in the presence of the reaction solution A mixed with guinea pig serum.
Comparative Example 4 - The labeled antibody used was normal rabbit IgG-GOD and the solid-phase antibody used was normal rabbit IgG. A normal rabbit IgG-coated polystyrene ball was incubated with the normal rabbit IgG-GOD in the presence of the reaction solution A mixed with guinea pig serum.

The results of the non-specific binding tests are illustrated in Table 1.

**TABLE 1**

| Example No. | Average Non-Specific Binding Percentage (%) | Comparative Example No. | Average Non-Specific Binding Percentage (%) |
|---|---|---|---|
| 1 | 0.015 | 1 | 0.078 |
| 2 | 0.017 | 2 | 0.190 |
| | | 3 | 0.115 |
| | | 4 | 0.148 |

The non-specific binding percentage ranges from 0.078% to 0.190% for combinations where the labeled antibody is originated from rabbit and the solid-phase antibody is originated from rabbit or where the labeled antibody is originated from goat and the solid-phase antibody is originated from goat. The non-specific binding percentage ranges from 0.015% to 0.017% for combinations where the labeled antibody is originated from rabbit and the solid-phase antibody is originated from goat. It can be seen from the test results that the degree of non-specific binding of the labeled antibody to the solid phase can be reduced to a remarkable extent when the labeled antibody is originated from rabbit and the solid-phase antibody is originated from goat.

The sensitivity and measurable range of such sandwich enzyme immunoassay was determined. For this purpose, an anti-AFP IgG-coated polystyrene ball was incubated at room temperature with 0.1 ml of AFP standard solution diluted with 0.2 ml of reaction solution A in an assay tube for six hours. The polystyrene ball was washed three times with the reaction solution A. The washed polystyrene ball was incubated with 0.1 ml of anti-AFP IgG-GOD labeled antibody diluted at a proper ratio with the reaction solution A. The assay tube was held stationary at room teperature all the night through. The polystyrene ball was then washed three times with the reaction solution A and transferred into another clean assay tube. Following this, 0.3 ml of 0.01 mol/l acetate buffer (pH 5.1) containing 0.5 mol/l glucose was added to the assay tube. The assay tube was held stationary at a temperature of 37°C for two hours. The resulting solution was taken to prepare a 0.1 ml sample. The sample was added with 0.5 ml of 0.2 mol/l carbonate buffer (pH 9.8) containing 2x10⁻⁷ mol/l luminol and then with 0.5 ml of 6x10⁻³ mol/l potassium ferricyanide. The light emitted upon chemiluminescent reaction of the luminol and potassium ferricyanide and the resulting hydrogen peroxide was measured by a Luminometer UPD-8000 which started its operation 15 seconds after the luminol and potassium ferricyanide were added to the assay tube and counted the light emitted for 30 seconds. Similar procedures are repeated for normal rabbit IgG antibodies and normal goat IgG antibodies.

The results of tests for the sandwich enzyme

**TABLE 2**

| Example No. | Number of Assay Repetitions | AFP (ng/ml) | Variation Coefficient (%) |
|---|---|---|---|
| | | Average Value ± Standard Deviation | |
| 1 | 10 | 11 ± 1.2 | 10.9 |
| 1 | 10 | 52 ± 4.3 | 8.3 |
| 1 | 10 | 295 ± 15.0 | 5.1 |

**TABLE 3**

| Comparative Example No. | Number of Assay Repetitions | AFP (ng/ml) | Variation Coefficient (%) |
|---|---|---|---|
| | | Average Value ± Standard Deviation | |
| 1 | 10 | 9 ± 1.9 | 21.8 |
| 1 | 10 | 49 ± 7.4 | 15.0 |
| 1 | 10 | 151 ± 21.9 | 14.5 |
| 2 | 10 | 13 ± 3.0 | 23.4 |
| 2 | 10 | 53 ± 9.3 | 17.5 |
| 2 | 10 | 155 ± 21.5 | 13.9 |

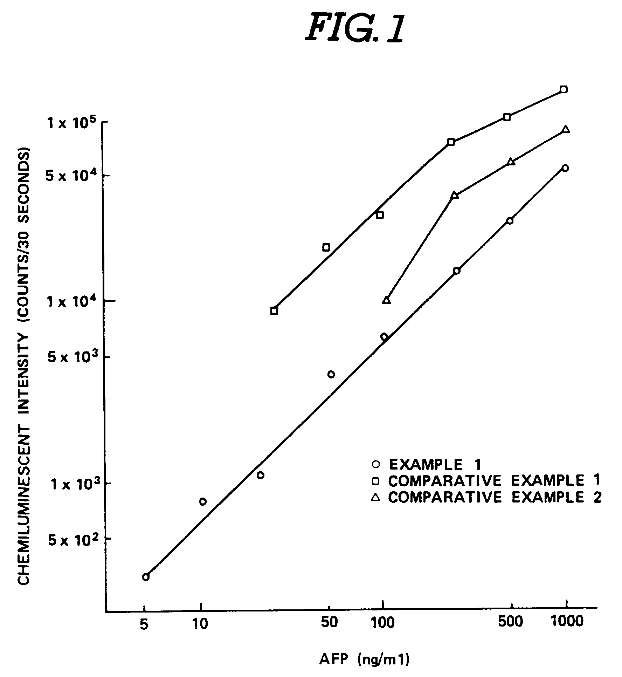

immunoassay sensitivity and measurable range are illustrated in Fig. 1. Fig. 1 relates to Example 1 and Comparative Examples 1 and 2. It can be seen from these test results that the sensitivity and measurable range of the sandwich enzyme immunoassay are superior when the labeled antibody is originated from rabbit and the solid-phase antibody is originated from goat to those obtained when the labeled antibody is originated from rabbit and the solid-phase antibody is originated from rabbit or when the labeled antibody is originated from goat and the solid-phase antibody is originated from goat.

Sandwich enzyme immunoassay was repeated ten times within a day (within assay) using the antibody in Example 1 and Comparative Examples 1 and 2. The results are shown in Tables 2 and 3. Similarly, sandwich enzyme immunoassay was repeated six times on different six days (between assay) using the antibody in Example 1 and Comparative Examples 1 and 2. The results are shown in Tables 4 and 5.

**TABLE 4**

| Example No. | Number of Assay Repetitions | AFP (ng/ml) | Variation Coefficient (%) |
|---|---|---|---|
| | | Average Value ± Standard Deviation | |
| 1 | 6 | 30 ± 2.1 | 7.1 |
| 1 | 6 | 142 ± 6.1 | 4.3 |

**TABLE 5**

| Comparative Example No. | Number of Assay Repetitions | AFP (ng/ml) | Variation Coefficient (%) |
|---|---|---|---|
| | | Average Value ± Standard Deviation | |
| 1 | 6 | 34 ± 8.5 | 25.0 |
| 1 | 6 | 136 ± 25.7 | 18.9 |
| 2 | 6 | 13 ± 2.5 | 18.9 |
| 2 | 6 | 125 ± 20.6 | 16.5 |

It can be seen from these results that smaller variations in the results of the sandwich enzyme immunoassay can be obtained when the labeled antibody is originated from rabbit and the solid-phase antibody is originated from goat than when the labeled antibody is originated from rabbit and the solid-phase antibody is originated from rabbit or when the labeled antibody is originated from goat and the solid-phase antibody is originated from goat.

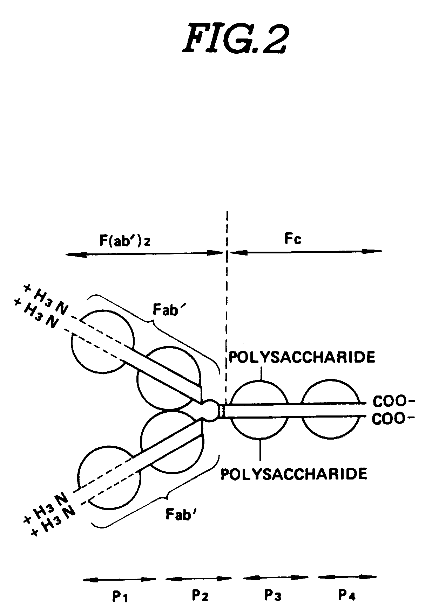

Referring to Fig. 2, there is illustrated in a schematic form the structure of Immunoglobulin G (IgG). The IgG structure can be divided into an antigen-binding F(ab')₂ fragment and a crystallizable (Fc) fragment when it is held at a temperature of 37°C in the presence of pepsin for 18 hours. The Fc fragment has a portion P4 for adsorption to a cell. The Fc fragment has no ability of binding to antigen and is crystallized at low temperature. The F(ab')₂ fragment can be divided under a reduction process, for example, in the presence of mercaptoethylamine, into two Fab' fragments having a portion P1 capable of binding to antigen. The inventors found that the degree of non-specific binding of the labeled antibody to the solid phase is dependent on the presence of the Fc fragment and can be reduced by replacing the IgG-GOD with Fab'-GOD when the solid-phase is originated from goat the labeled antibody is originated from rabbit. The Fab' fragment is prepared as the result of separation of the Fc fragment from IgG in the following manner:

First of all, 2 ml saturated ammonium sulfate is dripped into 2 ml of the serum drawn from a selected animal as described previously. The resulting solution is agitated slowly at a temperature of 4°C for two or three hours. The resulting cloudy solution is then placed in a centrifugation tube. Centrifugation is performed at a temperature of 4°C with a contrifugal separator rotating at a speed of 3000 rpm for 15 minutes. The supernatuant is removed from the centrifugation tube, whereas the precipitate is dissolved with 2 ml of 0.1 mol/l sodium phosphate buffer (pH 6.0) containing 5 mmol/l ethylenediaminetetraacetic acid (EDTA). The resulting solution is eluted at a flow rate of 6 ml/hour on a Sephacryl S-300 (a trademark of Pharmacia Fine Chemicals, Sweden) column (1 x 90 cm) equilibrated with 0.1 mol/l acetate buffer (pH 4.5) containing 0.1 mmol/l NaCI. The eluted solution is divided into 1 ml of samples. The lgG fractions, which absorb light at 280 nm are gathered from the respective samples. Following this, 0.1 ml of 3 mg/ml pepsin solution is added to the gathered IgG. The resulting solution is incubated at a temperature of 37°Cfor 18 hours. The resulting solution is eluted at a flow rate of 6 ml/hour on a Sephacryl S-200 (a trademark of Pharmacia Fine Chemicals, Sweden) column (1 x 90 cm) equilibrated with 0.1 mol/l sodium phosphate buffer (pH 7.5). The eluted solution is divided into 1 ml of samples. The F(ab')2 fractions, which absorb light at 280 nm, are gathered from the respective samples. The gathered F(ab')2 is added with 0.05 ml of 0.1 mol/l mercaptoethylamine and incubated at a temperature of 37°Cfor 90 minutes. The resulting solution is desalted at a flow rate of 6 ml/hour on a Sephadex G-25 (a trademark of Pharmacia Fine Chemicals, Sweden) column (1 x 30 cm) equilibrated with 0.1 mol/l sodium phosphate buffer (pH 6.0) containing 5 mmol/l EDTA. The desalted solution is divided into 1 ml of Fab' samples.

A number of tests were conducted substantially in the same manner as described previously to prove the advantageous effects on the non-specific binding of the Fab'-GOD labeled antibody to the solid phase.

Example 3 - The labeled antibody used was rabbit anti-AFP Fab'-GOD and the solid-phase antibody used was goat anti-AFP IgG. A goat anti-AFP IgG-coated polystyrene ball was incubated with the rabbit anti-AFP Fab'-GOD in the presence of the reaction solution A mixed with guinea pig serum.

Example 4 - The labeled antibody used was rabbit anti-CEA Fab'-GOD and the solid-phase antibody used was goat anti-CEA IgG. A goat anti-CEA IgG-coated polystyrene ball was incubated with the rabbit anti-CEA Fab'-GOD in the presence of the reaction solution A mixed with guinea pig serum.

For comparison of the non-specific binding reduction effect obtainable by the invention, tests were conducted for the following comparative examples:
Comparative Example 5 - The labeled antibody used was rabbit anti-AFP IgG-GOD and the solid-phase antibody used was goat anti-AFP IgG. A goat anti-AFP IgG-coated polystyrene ball was incubated with the rabbit anti-AFP IgG-GOD in the presence of the reaction solution A mixed with guinea pig serum.
Comparative Example 6 - The labeled antibody used was rabbit anti-AFP Fab'-GOD and the solid-phase antibody used was rabbit anti-AFP IgG. A rabbit anti-AFP IgG-coated polystyrene ball was incubated with the rabbit anti-AFP Fab'-GOD in the presence of the reaction solution A mixed with guinea pig serum.
Comparative Example 7 - The labeled antibody used was goat anti-AFP Fab'-GOD and the solid-phase antibody used was goat anti-AFP IgG. A goat anti-AFP IgG-coated polystyrene ball was incubated with the goat anti-AFP Fab'-GOD in the presence of the reaction solution A mixed with guinea pig serum.
Comparative Example 8 - The labeled antibody used was rabbit anti-CEA IgG-GOD and the solid-phase antibody used was goat anti-CEA IgG. A goat anti-CEA IgG-coated polystyrene ball was incubated with the rabbit anti-CEA IgG-GOD in the presence of the reaction solution A mixed with guinea pig serum.
Comparative Example 9 - The labeled antibody used was rabbit anti-CEA Fab'-GOD and the solid-phase antibody used was rabbit anti-CEA IgG. A rabbit anti-CEA IgG-coated polystyrene ball was incubated with the rabbit anti-CEA Fab'-GOD in the presence of the reaction solution A mixed with guinea pig serum.
Comparative Example 10 - The labeled antibody used was goat anti-CEA Fab'-GOD and the solid-phase antibody used was goat anti-CEA IgG. A goat anti-CEA IgG-coated polystyrene ball was incubated with the goat anti-CEA Fab'-GOD in the presence of the reaction solution A mixed with guinea pig serum.

The results of the non-specific binding tests are illustrated in Table 6.

**TABLE 6**

| Example No. | Average Non-Specific Binding Percentage (%) | Comparative Example No. | Average Non-Specific Binding Percentage (%) |
|---|---|---|---|
| 3 | 4.7 x 10⁻³ | 5 | 1.5 x 10⁻² |
| 4 | 3.8 x 10⁻³ | 6 | 2.3 x 10⁻² |
| | | 7 | 1.2 x 10⁻² |
| | | 8 | 1.3 x 10⁻² |
| | | 9 | 1.4 x 10⁻² |
| | | 10 | 1.8 x 10⁻² |

It can be seen from the test results that the degree of non-specific binding of the labeled antibody to the solid phase can be reduced to a remarkable extent when the labeled antibody is rabbit Fab' and the solid-phase antibody is goat IgG than when the labeled antibody is rabbit Fab' and the solid-phase antibody is rabbit IgG or when the labeled antibody is goat Fab' and the solid-phase antibody is goat IgG.

The measurable range and detection limit of sandwich enzyme immunoassay were determined substantially in the same manner as described previously. The results of tests for the sandwich enzyme immunoassay measurable range and detection limit are illustrated in Tables 7-10.

**TABLE 7**

| Example No. | Measurable Range AFP (ng/ml) | Comparative Example No. | Measurable Range AFP (ng/ml) |
|---|---|---|---|
| 3 | 0.1 - 50 | 5 | 1.0 - 100 |
| | | 6 | 2.5 - 100 |
| | | 7 | 1.0 - 100 |

**TABLE 8**

| Example No. | Measurable Range CEA (ng/ml) | Comparative Example No. | Measurable Range CEA (ng/ml) |
|---|---|---|---|
| 4 | 0.1 - 100 | 8 | 2.5 - 250 |
| | | 9 | 1.0 - 250 |
| | | 10 | 5.0 - 250 |

**TABLE 9**

| Example No. | Detection Limit AFP (ng/tube) | Comparative Example No. | Detection Limit AFP (ng/tube) |
|---|---|---|---|
| 3 | 0.01 | 5 | 0.1 |
| | | 6 | 0.25 |
| | | 7 | 0.1 |

**TABLE 10**

| Example No. | Detection Limit CEA (ng/tube) | Comparative Example No. | Detection Limit CEA (ng/tube) |
|---|---|---|---|
| 4 | 0.01 | 8 | 0.25 |
| | | 9 | 0.1 |
| | | 10 | 0.5 |

It can be seen from these test results that the measurable range and detection limit of sandwich enzyme immunoassay are superior when the labeled antibody used is Fab'-GOD to those obtained when the labeled antibody used is IgG-GOD. In addition, the measurable range and detection limit of sandwich enzyme immunoassay are superior when the labeled antibody is rabbit Fab' and the solid-phase antibody is goat IgG than when the labeled antibody is rabbit Fab' and the solid-phase antibody is rabbit IgG or when the labeled antibody is goat Fab' and the solid-phase antibody is goat IgG.

Sandwich enzyme immunoassay was repeated ten times within a day (within assay) using the antibodies in Examples 3-4 and Comparative Examples 5-10. The results are shown in Tables 11-14. Similarly, sandwich enzyme immunoassay was repeated six times on different six days (between assay) using the antibodies in Examples 3-4 and Comparative Examples 5-10. The results are shown in Tables 15-18.

**TABLE 11**

| Example No. | Number of Assay Repetitions | AFP (ng/ml) | Variation Coefficient (%) |
|---|---|---|---|
| | | Average Value ± Standard Deviation | |
| 3 | 10 | 2.7 ± 0.092 | 3.4 |

**TABLE 12**

| Comparative Example No. | Number of Assay Repetitions | AFP (ng/ml) | Variation Coefficient (%) |
|---|---|---|---|
| | | Average Value ± Standard Deviation | |
| 5 | 10 | 2.8 ± 0.15 | 5.3 |
| 6 | 10 | 2.9 ± 0.36 | 12.5 |
| 7 | 10 | 2.8 ± 0.39 | 13.9 |

**TABLE 13**

| Example No. | Number of Assay Repetitions | CEA (ng/ml) | Variation Coefficient (%) |
|---|---|---|---|
| | | Average Value ± Standard Deviation | |
| 4 | 10 | 5.2 ± 0.19 | 3.6 |

**TABLE 14**

| Comparative Example No. | Number of Assay Repetitions | CEA (ng/ml) | Variation Coefficient (%) |
|---|---|---|---|
| | | Average Value ± Standard Deviation | |
| 8 | 10 | 5.0 ± 0.16 | 3.2 |
| 9 | 10 | 5.2 ± 0.95 | 18.2 |
| 10 | 10 | 5.5 ± 0.91 | 16.5 |

**TABLE 15**

| Example No. | Number of Assay Repetitions | AFP (ng/ml) | Variation Coefficient (%) |
|---|---|---|---|
| | | Average Value ± Standard Deviation | |
| 3 | 6 | 2.6 ± 0.065 | 2.5 |

**TABLE 16**

| Comparative Example No. | Number of Assay Repetitions | AFP (ng/ml) | Variation Coefficient (%) |
|---|---|---|---|
| | | Average Value ± Standard Deviation | |
| 5 | 6 | 2.5 ± 0.12 | 4.8 |
| 6 | 6 | 2.4 ± 0.38 | 15.8 |
| 7 | 6 | 2.6 ± 0.48 | 18.6 |

**TABLE 17**

| Example No. | Number of Assay Repetitions | CEA (ng/ml) | Variation Coefficient (%) |
|---|---|---|---|
| | | Average Value ± Standard Deviation | |
| 4 | 6 | 5.5 ± 0.21 | 3.8 |

**TABLE 18**

| Comparative Example No. | Number of Assay Repetitions | CEA (ng/ml) | Variation Coefficient (%) |
|---|---|---|---|
| | | Average Value ± Standard Deviation | |
| 8 | 6 | 4.8 ± 0.24 | 5.0 |
| 9 | 6 | 5.3 ± 0.83 | 15.6 |
| 10 | 6 | 5.3 ± 0.78 | 14.8 |

It can be seen from these results that smaller variations in the results of the sandwich enzyme immunoassay can be obtained when the labeled antibody is rabbit Fab' and the solid-phase antibody is goat IgG.

The inventors also found that the degree of non-specific binding of the labeled antibody to the solid phase can be reduced by adding normal guinea pig serum to the reaction solution A in the present of which the labeled antibody binds to the solid-phase antibody through the antigen. A number of tests were conducted to prove the advantageous effect on the degree of non-specific binding of the labeled antibody to the solid phase.

Example 5 - The labeled antibody used was rabbit anti-AFP IgG-GOD and the solid-phase antibody used was goat anti-AFP IgG. The reaction solution used was a mixture of the reaction solution A and normal guinea pig serum. The volume ratio of the normal guinea pig serum to the reaction solution A was 1:5.

The degree of non-specific binding of the labeled antibody to the solid phase was determined. For this purpose, a goat anti-AFP IgG-coated polystyrene ball was incubated at room temperature with goat anti-AFP IgG dilluted with 0.3 ml of a reaction solution mixture X containing the reaction solution A (0.056 mol/l sodium phosphate buffer (pH 6.3) containing 0.1% sodium azide, 0.2% bovine serum albumin (BSA) and 0.337% NaCI) and normal guinea pig serum in an assay tube all the night through. The volume ratio of the normal guinea pig serum to the reaction solution A was 1:5. The polystyrene ball was washed three times with the reaction solution mixture X and then transferred into another clean assay tube. Following this, 0.3 ml of 0.01 mol/l acetate buffer (pH 5.1) containing 0.5 mol/l glucose was added to the assay tube. The assay tube was held stationary at a temperature of 37°C for two hours. The resulting solution was taken to prepare a 0.1 ml sample. The sample was added with 0.5 ml of 0.2 mol/l carbonate buffer (pH 9.8) containing 2 x 10⁻⁷ mol/l luminol and then with 0.5 ml of 6 x 10⁻³ mol/l potassium ferricyanide. The light emitted upon chemiluminescent reaction of the luminol and potassium ferricyanide and the resulting hydrogen peroxide was measured by a Luminometer UPD-8000 (a trademark of Meidensha Electric Mfg. Co. Japan) which starts its counting operation 15 second after the luminol and potassium ferricyanide were added to the assay tube and counted the light emitted for 30 seconds. The degree of non-specific binding of the labeled antibody to the solid phase was represented by the non-specific binding ratio given as C1/C2 x 100 (%) where C1 is the number of counts measured for the labeled antibody binding to the polystyrene ball and C2 is the number of counts corresponding to the whole amount of the labeled antibody used in the assay tube.

For comparison of the non-specific binding reduction obtainable by the invention, a test was conducted substantially in the same manner for the following comparative examples:
Comparative Example 11 - The labeled antibody used was rabbit anti-AFP IgG-GOD and the solid-phase antibody used was goat anti-AFP IgG. The reaction solution used was the reaction solution A mixed with normal rabbit serum. The volume ratio of the normal rabbit serum to the reaction solution A was 1:5.
Comparative Example 12 - The labeled antibody used was rabbit anti-AFP IgG-GOD and the solid-phase antibody used was goat anti-AFP IgG. The reaction solution used was the reaction solution A mixed with normal goat serum. The volume ratio of the normal goat serum to the reaction solution A was 1:5.
Comparative Example 13 - The labeled antibody used was rabbit anti-AFP IgG-GOD and the solid-phase antibody used was goat anti-AFP IgG. The reaction solution used was the reaction solution A mixed with normal horse serum. The volume ratio of the normal horse serum to the reaction solution A was 1:5.
Comparative Example 14 - The labeled antibody used was rabbit anti-AFP IgG-GOD and the solid-phase antibody used was goat anti-AFP IgG. The reaction solution used was the reaction solution A.

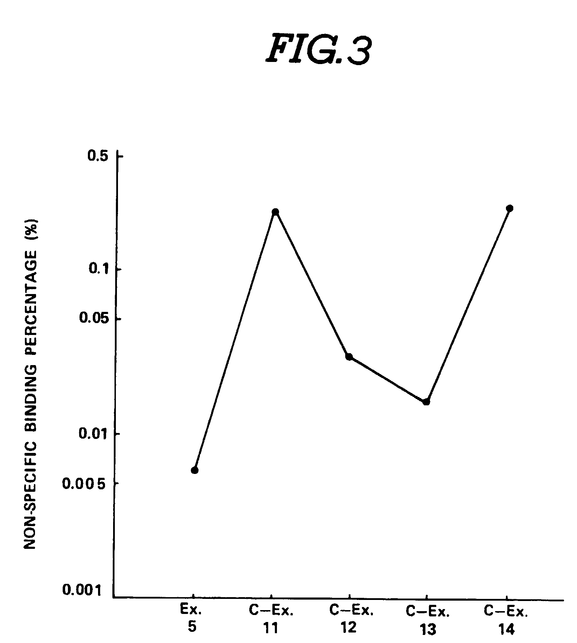

The results of the non-specific binding tests are illustrated in Fig. 3. The non-specific binding percentage was as small as 0.006% for Example 5. On the other hand for Comparative Examples 11-14, the non-specific binding percentage ranged from 0.016% to 0.25%. It is, therefore, apparent that the degree of non-specific binding of the labeled antibody to the solid phase can be reduced to a remarkable extent by adding normal guinea pig serum to the reaction solution.

The sensitivity and measurable range of sandwich enzyme immunoassay was determined for the following example:

Example 6 - The labeled antibody used was rabbit anti-AFP IgG-GOD and the solid-phase antibody used was goat anti-AFP IgG. The reaction solution used was a mixture of the raction solution A and normal guinea pig serum. The volume ratio of the normal guinea pig serum to the reaction solution A was 1:5.

A goat anti-AFP IgG-coated polystyrene ball was incubated at room temperature with AFP standard solution diluted with 0.3 ml of reaction solution A in an assay tube for six hours. The polystyrene ball was washed three times with the reaction solution A. The washed polystyrene ball was incubated with 0.1 ml of anti-AFP IgG-GOD labeled antibody diluted at a proper ratio with a mixture of the reaction solution A and normal guinea pig serum. The volume ratio of the normal guinea pig serum to the reaction solution A was 1:5. The assay tube was held stationary at room temperature all the night through. The polystyrene ball was then washed three times with the reaction solution A and transferred into another clean assay tube. Following this, 0.3 ml of 0.01 mol/l acetate buffer (pH 5.1) containing 0.5 mol/l glucose was added to the assay tube. The assay tube was held stationary at a temperature of 37°C for two hours. The resulting solution was taken to prepare a 0.1 ml sample. The sample was added with 0.5 ml of 0.2 mol/l carbonate buffer (pH 9.8) containing 2 x 10⁻⁷ mol/l luminol and then with 0.5 ml of 6 x 10⁻³ mol/l potassium ferricyanide. The light emitted upon chemiluminescent reaction of the luminol and potassium ferricyanide and the resulting hydrogen peroxide was measured by a Luminometer UPD-800 which started its operation 15 seconds after the luminol and potassium ferricyanide were added to the assay tube and counted the light emitted for 30 seconds.

Similar procedures were repeated for the following comparative example in order to prove the advantageous effects on the sandwich enzyme immunoassay:

Comparative Example 15 - The labeled antibody used was rabbit anti-AFP IgG-GOD and the solid-phase antibody used was goat anti-AFP IgG. The reaction solution used was the reaction solution A mixed with no normal guinea pig serum.

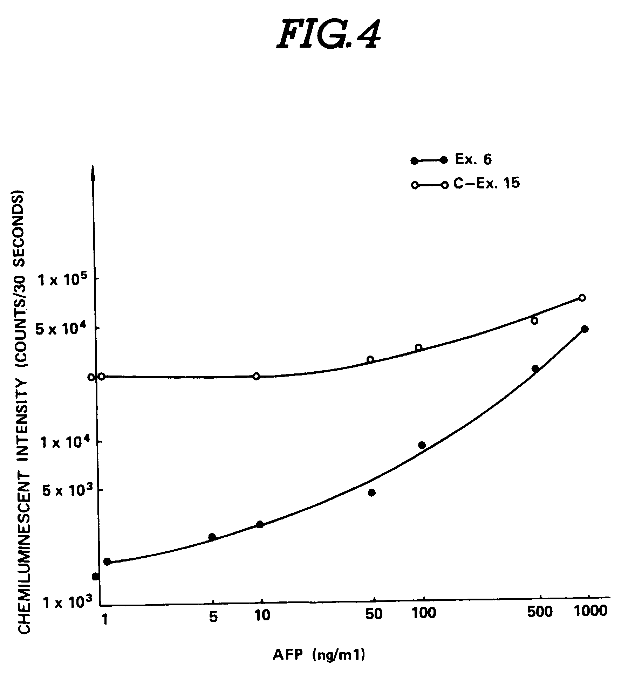

Fig. 4 illustrates the results. The sandwich enzyme immunoassay measurable range and sensitivity obtained for Example 6 were 1.0-1000 ng/ml and 1.0 ng/ml, respectively. On the other hand, the sandwich enzyme immunoassay measurable range and sensitivity were 50-1000 ng/ml and 50 ng/ml, respectively, for Comparative Example 15. It is, therefore, apparent that the sandwich enzyme immunoassay sensitivity and measurable range are improved when normal guinea pig serum is added to the reaction solution A.

Sandwich enzyme immunoassay was repeated ten times within a day (within assay) for Example 6 and Comparative Example 15. The results are shown in Tables 19 and 20. Similarly, sandwich enzyme immunoassay was repeated on different days (between assay) for Example 6 and Comparative Example 15. The results are shown in Tabels 21 and 22.

**TABLE 19**

| Example No. | Number of Assay Repetitions | AFP (ng/ml) | Variation Coefficient (%) |
|---|---|---|---|
| | | Average Value ± Standard Deviation | |
| 6 | 10 | 15 ± 1.2 | 7.8 |
| 6 | 10 | 123 ± 3.8 | 3.1 |
| 6 | 10 | 506 ± 14.7 | 2.9 |

**TABLE 20**

| Comparative Example No. | Number of Assay Repetitions | AFP (ng/ml) | Variation Coefficient (%) |
|---|---|---|---|
| | | Average Value ± Standard Deviation | |
| 15 | 10 | 60 ± 9.4 | 15.6 |
| 15 | 10 | 151 ± 16.9 | 11.2 |
| 15 | 10 | 615 ± 60.3 | 9.8 |

**TABLE 21**

| Example No. | Number of Assay Repetitions | AFP (ng/ml) | Variation Coefficient (%) |
|---|---|---|---|
| | | Average Value ± Standard Deviation | |
| 6 | 6 | 9.8 ± 6.5 | 6.6 |
| 6 | 6 | 62.7 ± 18.8 | 3.0 |

**TABLE 22**

| Comparative Example No. | Number of Assay Repetitions | AFP (ng/ml) | Variation Coefficient (%) |
|---|---|---|---|
| | | Average Value ± Standard Deviation | |
| 15 | 6 | 141 ± 18.8 | 13.3 |
| 15 | 6 | 708 ± 61.6 | 8.7 |

It can be seen from these results that smaller variations in the results of the sandwich enzyme immunoassay can be obtained when normal guinea pig serum is added to the reaction solution.

Although the reaction solution has been described as containing sodium phosphate buffer (pH 6.3), it is to be noted that it may be replaced with sodium phosphate buffer (pH 6.0-7.5), borate buffer (pH 8.0-8.5) or the like. In addition, although the invention has been described in connection with sandwich enzyme immunoassay, it is to be noted that the label may be a fluorescent material (fluorescein isothiocyanate, tetramethylrhodamine isothiocyanate. or the like), a chemiluminescent material (aminoethylethyl isoluminol, aminobutylethyl isoluminol, aminopentylethyl isoluminol, aminohexylethyl isoluminol, or the like), or a radioactive isotope (³H, ¹⁴C, ³²P, ¹²⁵I, ¹³¹I, or the like).

While the invention has been described in conjunction with specific embodiments thereof. it is intended to embrace all alternatives, modifications and variations that fall within the scope of the appended claims.

## Claims

1. A kit for use in a sandwich immunoassay employing labeled and solid-phase antibodies reactive with an antigen in the presence of a reaction solution to bind the antigen between the labeled and solid-phase antibodies, said kit containing a solid-phase antibody bound to a solid phase, a labeled antibody having a label enzyme bound to the antibody and a reaction solution, characterized in that the labeled antibody is originated from a rabbit, and the solid-phase antibody is originated from a goat and the label enzyme is glucose oxidase.

2. A kit as claimed in claim 1, wherein the labeled antibody is immunoglobulin G having a label enzyme bound thereto.

3. A kit as claimed in claim 1, wherein the labeled antibody is a Fab' fragment having a label enzyme bound thereto, the Fab' fragment being produced from immunoglobulin G.

4. A kit as claimed in claim 1, wherein the reaction solution contains normal guinea pig serum.

5. A kit as claimed in claim 4, wherein the labeled antibody is immunoglobulin G having a label enzyme bound thereto.

6. A kit as claimed in claim 5, wherein the labeled antibody is a Fab' fragment having a label enzyme bound thereto, the Fab' fragment being produced from immunoglobulin G.

## Patentansprüche

1. Kit zur Verwendung in einem Sandwich-Immunoassay, das markierte und Festphasen-Antikörper verwendet, die mit einem Antigen in Gegenwart einer Reaktionslösung reaktiv sind, um das Antigen zwischen dem markierten Antikörper und dem Festphasen-Antikörper zu binden, wobei das Kit einen Festphasen-Antikörper, der an eine feste Phase gebunden ist, einen markierten Antikörper, bei dem ein Markierungsenzym an den Antikörper gebunden ist, und eine Reaktionslösung enthält, dadurch gekennzeichnet, daß der markierte Antikörper von einem Kaninchen stammt, der Festphasen-Antikörper von einer Ziege stammt und das Markierungsenzym Glucoseoxidase ist.

2. Kit nach Anspruch 1, dadurch gekennzeichnet, daß der markierte Antikörper Immunglobulin G ist, an das das Markierungsenzym gebunden ist.

3. Kit nach Anspruch 1, dadurch gekennzeichnet, daß der markierte Antikörper ein Fab' Fragment ist, an das das Markierungsenzym gebunden ist, und daß das Fab' Fragment aus Immunglobulin G hergestellt ist.

4. Kit nach Anspruch 1, dadurch gekennzeichnet, daß die Reaktionslösung normales Meerschweinchenserum enthält.

5. Kit nach Anspruch 4, dadurch gekennzeichnet, daß der markierte Antikörper Immunglobulin G ist, an das das Markierungsenzym gebunden ist.

6. Kit nach Anspruch 5, dadurch gekennzeichnet, daß der markierte Antikörper ein Fab' Fragment ist, an das das Markierungsenzym gebunden ist, und daß das Fab' Fragment aus Immunglobulin G hergestellt ist.

## Revendications

1. Trousse pour utilisation dans un immuno-essai sandwich, utilisant des anticorps marqués et en phase solide qui réagissent avec un antigène en présence d'une solution de réaction pour lier l'antigène entre les anticorps marqués et en phase solide, ladite trousse contenant un anticorps en phase solide où l'anticorps est lié à une phase solide, un anticorps marqué où un enzyme de marquage est lié à l'anticorps, et une solution de réaction, caractérisée en ce que l'anticorps marqué provient d'un lapin, l'anticorps en phase solide provient d'une chèvre et l'enzyme de marquage est de la glucose oxydase.

2. Trousse selon la revendication 1, caractérisée en ce que l'anticorps marqué est de l'immunoglobuline G avec un enzyme de marquage qui lui est lié.

3. Trousse selon la revendication 1, caractérisée en ce que l'anticorps marqué est un fragment Fab' auquel est lié l'enzyme de marquage, le fragment Fab' étant produit à partir d'immunoglobuline G.

4. Trousse selon la revendication 1, caractérisée en ce que la solution de réaction contient du sérum normal de cobaye.

5. Trousse selon la revendication 4, caractérisée en ce que l'anticorps marqué est de l'immunoglobuline G avec un enzyme de marquage qui lui est lié.

6. Trousse selon la revendication 5, caractérisée en ce que l'anticorps marqué est un fragment Fab' auquel est lié l'enzyme de marquage, le fragment Fab' étant produit à partir d'immunoglobuline G.
